# EUROPEAN PATENT SPECIFICATION

(11) **EP 1 948 180 B1**
(45) Date of publication and mention of the grant of the patent: **13.03.2013**
(21) Application number: 06819380.4
(22) Date of filing: 09.11.2006
(51) Int. Cl.: A61K 31/517, A61K 31/00, A61P 35/00

(54) **COMBINATION TREATMENT OF CANCER COMPRISING EGFR/HER2 INHIBITORS**
KOMBINATIONSBEHANDLUNG GEGEN KREBS MIT EGFR/HER2-HEMMERN
TRAITEMENT COMBINE DU CANCER COMPRENANT DES INHIBITEURS DE EGFR/HER2

(30) Priority: 11.11.2005 EP 05110669
(43) Date of publication of application: 30.07.2008
(60) Divisional application: 11155582.7
(73) Proprietor: Boehringer Ingelheim International GmbH, 55216 Ingelheim am Rhein (DE); Boehringer Ingelheim Pharma GmbH & Co. KG, 55216 Ingelheim (DE)
(72) Inventor: SOLCA, Flavio, A-1230 Wien (AT); AMELSBERG, Andree, Southbury, CT 06488 (US); STEHLE, Gerd, 89584 Ehingen (DE); VAN MEEL, Jacobus C., A., A-2340 Moedling (AT); BAUM, Anke, A-1050 Wien (AT)
(74) Representative: Simon, Elke Anna Maria
(86) International application number: PCT/EP2006/068314
(87) International publication number: WO 2007/054551

(56) References cited:
- WO-A-02/50043
- WO-A1-2006/018182
- WO-A2-2004/096224
- RAMALINGAM SURESH ET AL: "Dual inhibition of the epidermal growth factor receptor with cetuximab, an IgG1 monoclonal antibody, and gefitinib, a tyrosine kinase inhibitor, in patients with refractory non-small cell lung cancer (NSCLC): a phase I study.", JOURNAL OF THORACIC ONCOLOGY : OFFICIAL PUBLICATION OF THE INTERNATIONAL ASSOCIATION FOR THE STUDY OF LUNG CANCER MAR 2008 LNKD- PUBMED:18317068, vol. 3, no. 3, March 2008 (2008-03), pages 258-264, ISSN: 1556-1380
- JANJIGIAN YELENA Y ET AL: "Phase I/II trial of cetuximab and erlotinib in patients with lung adenocarcinoma and acquired resistance to erlotinib.", CLINICAL CANCER RESEARCH : AN OFFICIAL JOURNAL OF THE AMERICAN ASSOCIATION FOR CANCER RESEARCH 15 APR 2011 LNKD- PUBMED:21248303, vol. 17, no. 8, 15 April 2011 (2011-04-15) , pages 2521-2527, ISSN: 1078-0432

## Description

The invention relates to a pharmaceutical composition comprising effective amounts of:
(1) as a compound 1
   4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline,
   or a pharmacologically acceptable acid addition salt thereof,
(2) and as a further chemotherapeutic agent 2 cetuximab.

### Background of the invention

Dual inhibitors of erbb1 receptor (EGFR) and erbB2 (Her2/neu) receptor tyrosine kinases are disclosed in WO 02/50043, WO 2004/074263 and WO 2005/037824, suitable for the treatment of e.g. benign or malignant tumours, particularly tumours of epithelial and neuroepithelial origin, metastasisation and the abnormal proliferation of vascular endothelial cells (neoangiogenesis), for treating diseases of the airways and lungs which are accompanied by increased or altered production of mucus caused by stimulation by tyrosine kinases, as well as for treating diseases of the gastrointestinal tract and bile duct and gall bladder which are associated with disrupted activity of the tyrosine kinases. The disclosure of WO 02/50043, WO 2004/074263 and WO 2005/037824 includes preparation as well as pharmaceutical formulations of the compounds. Furthermore, it is known for treatment of tumour diseases that the compounds may be used in monotherapy or in conjunction with other anti-tumour therapeutic agents, for example in combination with topoisomerase inhibitors (e.g. etoposide), mitosis inhibitors (e.g. vinblastine), compounds which interact with nucleic acids (e.g. cis-platin, cyclophosphamide, adriamycin), hormone antagonists (e.g. tamoxifen), inhibitors of metabolic processes (e.g. 5-FU etc.), cytokines (e.g. interferons) or antibodies. Treatment of tumour diseases with the combination of the VEGFR inhibitor 3-Z-[1-(4-(N-((4-methyl-piperazin-1-yl)-methylcarbonyl)-N-methyl-amino)-anilino)-1-phenyl-methylene]-6-methoxycarbonyl-2-indolinone and one of the dual EGFR/HER2 inhibitors 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline or 4-[(3-chloro-4-fluoro-phenyl)amino]-6- {[4-(homomorpholin-4-yl)-1-oxo-2-buten-1-yl]amino}-7-[(S)-(tetrahydrofuran-3-yl)oxy]-quinazoline are disclosed in WO 2004/096224.

For the treatment of diseases of oncological nature, a large number of chemotherapeutic, immunotherapeutic or immunomodulatory, antiangiogenic or hormonal agents have already been suggested, which can be used as monotherapy (treatment with one agent) or as combination therapy (simultaneous, separate or sequential treatment with more than one agent) and/or which may be combined with radiotherapy or radio-immunotherapy. In this respect, chemotherapeutic agent means a naturally occurring, semi-synthetic or synthetic chemical compound which, alone or via further activation, for example with radiations in the case of radio-immunotherapy, inhibits or kills growing cells, and which can be used or is approved for use in the treatment of diseases of oncological nature, which are commonly also denominated as cancers. In the literature, these agents are generally classified according to their mechanism of action. In this matter, reference can be made, for example, to the classification made in "Cancer Chemotherapeutic Agents", American Chemical Society, 1995, W.O. Foye Ed.

The efficacy of chemotherapeutic agents can be improved by using combination therapies with other chemotherapeutic, immunotherapeutic, immunomodulatory, antiangiogenic or hormonal compounds. Combination therapies constitute the gold standard in many settings of cancer therapy.

Even if the concept of combining several therapeutic agents or therapies already has been suggested, and although various combination therapies are under investigation and in clinical trials, there is still a need for new and efficient therapeutic compositions for the treatment of cancer diseases, which show advantages over standard therapies. It is the purpose of the present invention to provide a combination therapy with a dual inhibitor for the treatment of various cancer diseases.

### Summary of the Invention

It has been found that a combination therapy for treatment of various cancer diseases, especially of the specific cancer-subindications mentioned hereinafter, comprising coadministration to a patient and/or co-treatment of a patient with effective amounts of:
(1) as a compound 1
   4-[(3-chloro-4-fluorophenyl)amino]-6- {[4-(N,N-dimethylamino)-1-oxo-2-buten-1-yl]amino}-7-((S)-tetrahydrofuran-3-yloxy)-quinazoline, or a pharmacologically acceptable acid addition salt thereof,
(2) and as a further chemotherapeutic agent **2** cetuximab;
optionally in combination with radiotherapy, radio-immunotherapy and/or tumour resection by surgery,
provides unexpected advantages, e.g. superior efficacy based on additive or synergistic effects and/or improved tolerability and reduced side effects of the treatment by the patient due, for example, to the administration of lower doses of the therapeutic agents involved reduced side effects.

Any reference to compound **1** in connection with the invention should be understood to include the pharmacologically acceptable acid addition salts, solvates, hydrates, or polymorphs thereof.

The expression "patient" relates to a human or non-human mammalian patient suffering from cancer and thus in need of such treatment, preferably the patient is a human person. Furthermore, the expression "patient" should be understood to include such cancer patients carrying tumors with wild-type EGF receptor as well as pre-selected cancer patients with tumors harboring activating EGFR mutations. These can be located in the tyrosine kinase domain of the EGF receptor such as for instance the L858R or L861 point mutations in the activation loop (exon 21), or in-frame deletion/insertion mutations in the ELREA sequence (exon 19), or substitutions in G719 situated in the nucleotide binding loop (exon 18). Additional activating mutations have been reported in the extracellular domain of the EGF receptor in various indications (e.g. EGFR vIII displaying exon 2-7 deletions). Other mutations such as the T790M point mutation in exon 20 as well as certain exon 20 insertions (e.g. D770_N771insNPG) which confer resistance to particular drugs should also be included, as well as double mutants such as the combined L858R / T790M mutation or the exon-19-del/T790M.

The expression "patient" should be understood to include also such cancer patients carrying tumors with wild-type HER2 receptor as well as pre-selected cancer patients with tumors harboring activating HER2 mutations, e.g. M774_A775insAYVM.

The indication "cancer" as used in the context of the invention is to be understood in a most general sense as a disease characterized by inappropriate cellular proliferation, migration, apoptosis or angiogenesis, preferably by inappropriate cellular proliferation. Inappropriate cell proliferation means cellular proliferation resulting from inappropriate cell growth, from excessive cell division, from cell division at an accelerated rate and/or from inappropriate cell survival.

"Radiotherapy" means administering ionizing radiation to the patient, as conventionally used in cancer therapy. Radiotherapy may be applied before, in parallel or after co-treatment by administration of the actives **1** and **2.**

"Tumour resection by surgery" is one standard option in cancer therapy and may be applied before or after co-treatment by administration of the actives **1** and **2.**

A first aspect of the present invention relates to a pharmaceutical composition for the treatment of cancer comprising effective amounts of:
(1) as a compound **1**
   4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline,
   or a pharmacologically acceptable acid addition salt thereof; and
(2) as a further chemotherapeutic agent **2** cetuximab;
optionally in combination with one or more pharmaceutically acceptable excipients, and optionally adapted for a co-treatment with radiotherapy or radio-immunotherapy, in the form of a combined preparation for simultaneous, separate or sequential use in the treatment of diseases involving cell proliferation, migration or apoptosis of cancer cells, or angiogenesis, preferably involving cell proliferation or apoptosis of cancer cells.

A second aspect of the present invention is directed to the use of compound **1**, 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline, for the manufacture of a pharmaceutical composition for the treatment of the specific cancer-subindications referred to hereinafter, comprising effective amounts of:
(1) as a compound **1** 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline,
   or a pharmacologically acceptable acid addition salt thereof; and
(2) as a further chemotherapeutic agent 2 cetuximab;
optionally in combination with one or more pharmaceutically acceptable excipients, and optionally adapted for a co-treatment with radiotherapy or radio-immunotherapy, in the form of a combined preparation for simultaneous, separate or sequential use.

The expression "a pharmaceutical composition for the treatment of cancer" should be understood interchangeable with "a medicament for the treatment of cancer".

### Detailed Description of the Invention

In one embodiment, with regard to any aspect of the invention, compound **1** is selected from the group consisting of
4-[(3-chloro-4-fluorophenyl)amino]-6- {[4-(N,N-dimethylamino)-1-oxo-2-buten-1-yl]amino}-7-((S)-tetrahydrofuran-3-yloxy)-quinazoline, 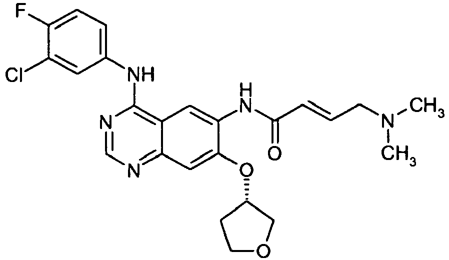 and
4-[(3-chloro-4-fluorophenyl)amino]-6-{[4-(N,N-dimethylamino)-1-oxo-2-buten-1-yl]-amino}-7-((S)-tetrahydrofuran-3-yloxy)-quinazoline dimaleate, and
the chemotherapeutic agent 2 is cetuximab.

In a preferred embodiment **B,** with regard to the first and second aspect of the invention, compound **1** is
4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline (BIBW2992),
the chemotherapeutic agent 2 is cetuximab,
and the cancer indication is selected from the group consisting of
o Head and neck tumours: SCC, AC, transitional cell cancers, mucoepidermoid cancers, undifferentiated carcinomas;
o Colorectal cancers, metastatic or non-metastatic: AC, including hereditary forms of AC, carcinoid, sarcoma;
o Pancreatic cancers: AC, including ductal and acinary cancers, papillary, adenosquamous, undifferentiated, tumours of the endocrine pancreas;
o Breast cancers, metastatic or non-metastatic: AC, including invasive ductal, lobular and medullary cancers, tubular, mucinous cancers, Paget-carcinoma, inflammatory carcinoma, ductal and lobular carcinoma in situ;
o Prostate cancers: AC, small cell, SCC;
o Gastric cancers: AC, adenosquamous, anaplastic;
o Ovarian cancer;
o Non-small cell lung cancers (NSCLC): SCC, spindle cell carcinoma, AC, bronchioalveolar carcinoma, large cell NSCLC, clear cell NSCLC.

In a preferred embodiment **C**, with regard to the first and second aspect of the invention, compound 1 is
4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline (BIBW2992),
the chemotherapeutic agent **2** is cetuximab,
and the cancer indication is selected from the group consisting of
o Head and neck tumours: SCC, AC, transitional cell cancers, mucoepidermoid cancers, undifferentiated carcinomas;
o Colorectal cancers, metastatic or non-metastatic: AC, including hereditary forms of AC, carcinoid, sarcoma;
o Pancreatic cancers: AC, including ductal and acinary cancers, papillary, adenosquamous, undifferentiated, tumours of the endocrine pancreas;
o Breast cancers, metastatic or non-metastatic: AC, including invasive ductal, lobular and medullary cancers, tubular, mucinous cancers, Paget-carcinoma, inflammatory carcinoma, ductal and lobular carcinoma in situ;
o Prostate cancers: AC, small cell, SCC;
o Non-small cell lung cancers (NSCLC): SCC, spindle cell carcinoma, AC, bronchioalveolar carcinoma, large cell NSCLC, clear cell NSCLC.

In a preferred embodiment **D**, with regard to the first and second aspect of the invention, compound **1** is
4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline (BIBW2992) or a pharmacologically acceptable salt thereof, preferably the dimaleate salt,
the chemotherapeutic agent **2** is cetuximab,
and the cancer indication is selected from the group consisting of
o Head and neck tumours: SCC, AC, transitional cell cancers, mucoepidermoid cancers, undifferentiated carcinomas;
o Colorectal cancers, metastatic or non-metastatic: AC, including hereditary forms of AC, carcinoid, sarcoma;
o Pancreatic cancers: AC, including ductal and acinary cancers, papillary, adenosquamous, undifferentiated, tumours of the endocrine pancreas;
o Breast cancers, metastatic or non-metastatic: AC, including invasive ductal, lobular and medullary cancers, tubular, mucinous cancers, Paget-carcinoma, inflammatory carcinoma, ductal and lobular carcinoma in situ;
o Prostate cancers: AC, small cell, SCC;
o Non-small cell lung cancers (NSCLC): SCC, spindle cell carcinoma, AC, bronchioalveolar carcinoma, large cell NSCLC, clear cell NSCLC.

It is known that cancer patients carrying activating EGFR mutations in their tumors, i. e. within the tyrosine kinase domain of the EGF receptor, may show increased sensitivity to treatment with EGFR inhibitors. Analogously, cancer patients carrying activating HER2 mutations, e.g. M774_A775insAYVM, in their tumors may show increased sensitivity to treatment with HER2 inhibitors. Both groups of patients as well as a subgroup carrying both activating EGFR and HER2 mutations may show increased sensitivity to treatment with dual inhibitors of erbb1 receptor (EGFR) and erbB2 (Her2/neu).

The presence of specific gain-of-function mutations within the tyrosine kinase domain of the EGF receptor in a subgroup of NSCLC patients has been associated with increased sensitivity to treatment with gefitinib and erlotinib (Lynch, New England Journal Medicine 350, 2129 (2004); Paez, Science 304, 1497 (2004); Pao, Proceedings of the National Academy of Science of the United States 101, 13306 (2004)). In particular, the L858R point mutation (exon 21) as well as deletion/insertion mutations in the ELREA sequence (exon 19) account for the majority of gefitinib responders. A secondary point mutation in exon 20, T790M, is associated with acquired resistance to gefitinib or erlotinib. This mutation is analogous to the T315I mutation identified in CML patients who relapse under imatinib treatment (imatinib resistant patients).

Irreversible inhibitors (e.g., HKI-272 or CL 387,785), in contrast to reversible inhibitors (e.g., gefitinib), are able to inhibit proliferation and EGF-induced EGFR phosphorylation in cell lines expressing double mutant EGF receptors (Kwak, Proceedings of the National Academy of Science of the United States 102, 7665 (2005) and Kobayashi, New England Journal Medicine 352, 786 (2005)).

For the therapeutic use of a pharmaceutical composition of the invention pre-selection of cancer patients for an EGFR mutation in the tyrosine kinase domain of the EGF receptor as well as pre-selection of cancer patients for an HER2 mutation may be relevant. The EGFR mutations preferably relevant in in this context are selected from the group consisting of the L858R and L861 point mutations in the activation loop (exon 21), in-frame deletion/insertion mutations in the ELREA sequence (exon 19), substitutions in G719 situated in the nucleotide binding loop (exon 18), activating mutations in the extracellular domain of the EGF receptor such as EGFR vIII displaying exon 2-7 deletions, the T790M point mutation in exon 20, exon 20 insertions such as D770_N771insNPG, and double mutants such as the combined L858R /T790M mutation and the exon-19-del/T790M. The HER2 mutation preferably relevant in in this context is the M774_A775insAYVM mutation.

Methods for detecting mutations in the tyrosine kinase domain of the EGF receptor are kown in the art, several corresponding diagnostic tools are approved by the FDA and commercially available, e.g. an assay for the detection of epidermal growth factor receptor mutations in patients with non-small cell lung cancer (Genzyme Corp.; see also Journal of Clinical Oncology, 2006 ASCO Annual Meeting Proceedings (Post-Meeting Edition). Vol 24, No 18S (June 20 Supplement), 2006: Abstract 10060).

Most important cancer indications with EGFR or HER2 mutations relevant in connection with patient pre-selection for mutations are selected from the group consisting of
o Head and neck tumours: SCC, AC, transitional cell cancers, mucoepidermoid cancers, undifferentiated carcinomas;
o Colorectal cancers, metastatic or non-metastatic: AC, including hereditary forms of AC, carcinoid, sarcoma;
o Pancreatic cancers: AC, including ductal and acinary cancers, papillary, adenosquamous, undifferentiated, tumours of the endocrine pancreas;
o Breast cancers, metastatic or non-metastatic: AC, including invasive ductal, lobular and medullary cancers, tubular, mucinous cancers, Paget-carcinoma, inflammatory carcinoma, ductal and lobular carcinoma in situ;
o Prostate cancers: AC, small cell, SCC;
o Gastric cancers: AC, adenosquamous, anaplastic;
o Ovarian cancer;
o Non-small cell lung cancers (NSCLC): SCC, spindle cell carcinoma, AC, bronchioalveolar carcinoma, large cell NSCLC, clear cell NSCLC, but especially
o Non-small cell lung cancers (NSCLC): SCC, spindle cell carcinoma, AC, bronchioalveolar carcinoma, large cell NSCLC, clear cell NSCLC, especially metastatic, second line patients who have failed at least one prior chemotherapy regimen or 3rd/4th line patients who have received Tarceva^{®} or Iressa^{®} for at least 12 weeks and then failed.

### Method of treatment:

The pharmaceutical compositions according to the invention are suitable in a method for the treatment of cancer, which method comprises simultaneous, separate or sequential coadministration of effective amounts of:
(1) as a compound **1**
   4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline
   or a pharmacologically acceptable acid addition salt thereof; and
(2) as a further chemotherapeutic agent **2** cetuximab;
in the form of a combined preparation optionally adapted for a co-treatment with radiotherapy or radio-immunotherapy, to a person in need of such treatment.

The term "therapeutically effective amount" shall mean that amount of a drug or pharmaceutical agent that will elicit the biological or medical response of a tissue, system, animal or human that is being sought by a researcher or clinician.

In accordance with the present invention, the elements of the combination of **1** and **2** may be administered by oral (including buccal or sublingual), enterical, parenteral (e.g., intramuscular, intraperitoneal, intravenous, transdermal or subcutaneous injection, or implant), nasal, vaginal, rectal, or topical (e.g. inhalative) routes of administration and may be formulated, alone or together, in suitable dosage unit formulations containing conventional non-toxic pharmaceutically acceptable carriers, adjuvants and vehicles appropriate for each route of administration.

In a preferred embodiment the element **1** of the combination in accordance with the invention is administered orally, enterically, transdermally, intravenously, peritoneally or by injection, preferably orally.

### Dosages / compound 1:

In the method of treatment described above, characterised in that compound **1**, or its polymorph, hydrate, solvate, or a pharmaceutically acceptable salt thereof, is administered intermittent or in a daily dosage such that the plasma level of the active substance preferably lies between 10 and 5000 nM for at least 12 hours of the dosing interval.

The compound **1** may be administered to the human patient in a daily dose of 0.01-4 mg/kg of body weight (bw), preferably 0.1-2 mg/kg, particularly preferred in a dose of 0.2-1.3 mg/kg bw. For oral treatment compound**1** may be administered daily in a total dose of 10, 20, 30, 40, 50, 60, 70, 100, 200, or 300 mg, optionally divided into multiple doses, e.g. 1 to 3 doses to be administered through the day. Preferably the oral daily dose is administered only once a time. These doses can be applied with compound **1** BIBW2992 or an equivalent dose of BIBW2992MA₂ containing respective amounts of the active base component. Especially for higher doses periods of treatment should alternate with periods of recovery, without administering the active of formula (I). For instance, treatment could follow a "7 day on - 7 day off", a "14 day on - 14 day off', a "21 day on 7 day off" or a continuous dosing schedule. "On-off' time periods can be chosen shorter, especially if higher doses are administered, or individually adapted to the needs of the patient.

The dosage for intravenous use of BIBW2992MA₂ may be 1 - 300 mg, particularly preferred 10 - 100 mg (dosages refer to the base form BIBW2992), either given as a bolus or, especially if higher doses are applied, as a slow intravenous infusion over several hours, e.g. over about 1, 2, 4, 6, 10, 12 or 24 hours.

However, it may optionally be necessary to deviate from the amounts specified, depending on the body weight or method of administration, the individual response to the medication, the nature of the formulation used and the time or interval over which it is administered. Thus, in some cases, it may be sufficient to use less than the minimum quantity specified above, while in other cases the upper limit specified will have to be exceeded. When large amounts are administered it may be advisable to spread them over the day in a number of single doses.

### Dosages / chemotherapeutic agent 2:

Dosages and treatment schedules for chemotherapeutic agent **2** are known in the art and may be applied analogously within the invention. Dosage of the chemotherapeutic agent **2** may be reduced, e.g. may vary in the range of 1/1 to 1/20 of the dosages described in the prior art.

However, it may optionally be necessary to deviate from the amounts specified, depending on the body weight or method of administration, the individual response to the medication, the nature of the formulation used and the time or interval over which it is administered. Thus, in some cases, it may be sufficient to use less than the minimum quantity specified above, while in other cases the upper limit specified will have to be exceeded. When large amounts are administered it may be advisable to spread them over the day in a number of single doses.

### Dosages / radiotherapy or radio-immunotherapy:

Dosages and treatment schedules for radiotherapy and radio-immunotherapy are known in the art and may be applied analogouslyin conjunction with the methods of treatment described hereinbefore. The dosage of the radiotherapy and radio-immunotherapy component may be reduced, e.g. may vary in the range of 1/1 to 1/20 of the dosages described in the prior art.

### Pharmaceutical compositions:

As used herein, the term "composition" is intended to encompass a product comprising the specified ingredients in the specified amounts, as well as any product which results, directly or indirectly, from a combination of the specified ingredients in the specified amounts. The amount of pharmaceutically active compound in each case should be in the range from 0.1 - 90 wt.%, preferably 0.5 - 50 wt.% of the total composition, i.e. in amounts which are sufficient to achieve the dosage ranges given hereinbefore. The doses specified may, if necessary, be given several times a day.

As already mentioned before, the components **1** and **2** of the composition for a combination therapy may be administered separately (which implies that they are formulated separately) or together. Hence, the administration of one element of the combination of the present invention may be prior to, concurrent to, or subsequent to the administration of the other element of the combination.

One embodiment of the invention relates to a pharmaceutical combination preparation kit for the treatment of cancer diseases, comprising
(i) a first compartment containing a pharmaceutical composition comprising a therapeutically effective amount of compound **1**,
   4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline,
   or a pharmacologically acceptable acid addition salt thereof, and
(ii) a second containment containing a pharmaceutical composition comprising as a chemotherapeutic agent **2** cetuximab in a therapeutically effective amount,
said kit being optionally adapted for a co-treatment with radiotherapy or radio-immunotherapy.

In a preferred embodiment the invention relates to a pharmaceutical combination preparation kit, wherein the formulation of the compound **1** in accordance with the present invention is for oral administration.

The pharmaceutical compositions for the administration of the components **1** and **2** of this invention may conveniently be presented in dosage unit form and may be prepared by any of the methods well known in the art of pharmacy. All methods include the step of bringing the active ingredient into association with the carrier which is constituted of one or more accessory ingredients. In general, the pharmaceutical compositions are prepared by uniformly and intimately bringing the active ingredients into association with a liquid carrier or a finely divided solid carrier or both, and then, if necessary, shaping the product into the desired dosage form. In the pharmaceutical compositions the active compounds are included in an amount sufficient to produce the desired pharmacologic effect.

Suitable excipients may be, for example, water, pharmaceutically acceptable organic solvents, such as paraffins (e.g. petroleum fractions), oils of vegetable origin (e.g. groundnut or sesame oil), mono- or polyfunctional alcohols (e.g. ethanol or glycerol), carriers such as e.g. natural mineral powders (e.g. kaolin, clays, talc, chalk), synthetic mineral powders (e.g. highly dispersed silica and silicates), sugar (e.g. glucose, lactose and dextrose), emulsifiers (e.g. lignin, spent sulphite liquors, methylcellulose, starch and polyvinylpyrrolidone) and lubricants (e.g. magnesium stearate, talc, stearic acid and sodium lauryl sulphate).

The preparations are administered in the usual way, preferably by oral or transdermal route, particularly preferably by oral route. When administered orally the tablets may, of course, contain additives, such as e.g. sodium citrate, calcium carbonate and dicalcium phosphate together with various additives, such as starch, preferably potato starch, gelatine and the like, in addition to the abovementioned carriers. Lubricants such as magnesium stearate, sodium laurylsulphate and talc may also be used to form tablets. In the case of aqueous suspensions the active substances may be combined with various flavour enhancers or colourings in addition to the abovementioned excipients.

For parenteral use, solutions of the active substances may be prepared using suitable liquid carrier materials.

Dosage forms intended for oral use may be prepared according to any method known to the art for the manufacture of pharmaceutical formulations and such compositions. The excipients used may be, for example: (a) inert diluents such as mannitol, sorbitol, calcium carbonate, pregelatinized starch, lactose, calcium phosphate or sodium phosphate; (b) granulating and disintegrating agents, such as povidone, copovidone, hydroxypropylmethylcellulose, corn starch, alginic acid, crospovidone, sodiumstarchglycolate, croscarmellose, or polacrilin potassium; (c) binding agents such as microcrystalline cellulose or acacia; and (d) lubricating agents such as magnesium stearate, stearic acid, fumaric acid or talc.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

Tablets, capsules or pellets may be uncoated or they may be coated by known techniques to delay disintegration and absorption in the gastrointestinal tract and thereby provide a delayed action or sustained action over a longer period. For example, a time delay material such as celluloseacetate phtalate or hydroxypropylcellulose acetate succinate or sustained release material such as ethylcellulose or ammoniomethacrylate copolymer (type B) may be employed.

Liquid dosage forms for oral administration in accordance with the present invention include pharmaceutically acceptable emulsions, solutions, suspensions, syrups, and elixirs containing inert diluents commonly used in the art, such as water. Besides such inert diluents, compositions can also include adjuvants, such as wetting agents, emulsifying and suspending agents, and sweetening, flavoring, perfuming and preserving agents.

Syrups or elixirs containing the active substances or combinations thereof according to the invention may additionally contain a sweetener such as saccharin, cyclamate, glycerol or sugar and a flavour enhancer, e.g. a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

The pharmaceutical compositions containing **1** and **2,** separately or together, may be in the form of a sterile injectable aqueous or oleagenous suspension or solution. The suspension may be formulated according to known methods using those suitable dispersing or wetting agents and suspending agents which have been mentioned hereinbefore. A suitable sterile injectable preparation may also be a sterile injectable solution or suspension in a non toxic parenterally-acceptable diluent or solvent, for example a solution in 1,3-butane-diol. Examples of suitable acceptable vehicles and solvents that may be employed are water, Ringer's solution and an isotonic sodium chloride solution. In addition, sterile, fixed oils may conventionally be employed as a solvent or suspending medium. For this purpose any bland fixed oil may be employed, including synthetic mono-or diglycerides. In addition, fatty acids such as oleic acid find use in the preparation of injectables in accordance with the present invention. Preparations for parenteral administration according to the present invention containing 1 and 2, separately or together, include sterile aqueous or non-aqueous solutions, suspension, or emulsions.

Solutions for injection and infusion are prepared in the usual way, e.g. with the addition of preservatives such as p-hydroxybenzoates, or stabilisers such as alkali metal salts of ethylenediamine tetraacetic acid, optionally using emulsifiers and/or dispersants, while if water is used as the diluent organic solvents may optionally be used as solubilisers or auxiliary solvents, and transferred into injection vials or ampoules or infusion bottles.

Examples of suitables non-aqueous solvents or vehicles for the preparations in accordance with the present invention are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, by filtration through a bacteria-retaining filter, by incorporating sterilizing agents into the compositions, by irradiating the compositions, or by heating the compositions. They may also be manufactured in the form of sterile solid compositions which can be reconstituted in sterile water, or some other sterile injectable medium immediately before use.

Compositions for buccal, nasal or sublingual administration in accordance with the present invention may be prepared with standard excipients well known in the art.

The dosage of the active ingredients in the compositions in accordance with the present invention may be varied, although the amount of the active ingredients **1** and **2** shall be such that a suitable dosage form is obtained. Hence, the selected dosage and the selected dosage form shall depend on the desired therapeutic effect, the route of administration and the duration of the treatment. Suitable dosage ranges for the combination are from the maximal tolerated dose for the single agent to lower doses, e.g. to one tenth of the maximal tolerated dose.

The following Examples 1 to 6 serve to illustrate the invention without restricting it. The Examples contain as active substance compound **1**.

### Example 1: Coated immediate-release tablets containing 75 mg of active substance by dry-granulation process

### Composition:

1 tablet contains:

| | |
|---|---|
| active substance | 75.0 mg |
| calcium phosphate anhydrous | 108.0 mg |
| corn starch | 35.5 mg |
| polyvinylpyrrolidone | 10.0 mg |
| magnesium stearate | 1.5 mg |
| hydroxypropylmethylcellulose | 7.5 mg |
| polyethylene glycol | 1.0 mg |
| polydextrose | 5.0 mg |
| talc | 1.0 mg |
| pigments | 0.5 mg |
| water (volatile) | *** |
| | 245.0 mg |

### Preparation:

The active substance is mixed with calcium phosphate, corn starch, polyvinylpyrrolidone, hydroxypropylmethylcellulose and half the specified amount of magnesium stearate. Ribbons are produced in a roller-compactor and these are then rubbed through a screen with a mesh size of 1.5 mm using a suitable machine and mixed with the rest of the magnesium stearate. This granulate is compressed in a tablet-making machine to form tablets of the desired shape.

| | |
|---|---|
| Weight of core: | 230 mg |
| Tablet shape: | 9 mm round, bi- convex |

The tablet cores are subsequently coated with an aqueous film-coat consisting essentially of hydroxypropylmethylcellulose, polyethylene glycol, polydextrose, talc and pigments.
Weight of coated tablet: 245 mg.

### Example 2: Extended- release tablets containing 100 mg of active substance by organic granulation granulation process

| 1 tablet contains: | |
|---|---|
| active substance | 100.0 mg |
| lactose | 34.0 mg |
| hydroxypropylmethylcellulose | 80 mg |
| polyvinylpyrrolidone | 4.0 mg |
| magnesium stearate | 2.0 mg |
| ethanol (volatile) | *** |
| | 220.0 mg |

### Preparation:

The active substance, lactose and hydroxypropylmethylcellulose are mixed together and uniformly moistened with solution of the polyvinylpyrrolidone in ethanol. After the moist composition has been screened (2.0 mm mesh size) and dried in a rack-type drier at 50°C it is screened again (1.5 mm mesh size) and the lubricant is added. The final blend is compressed to form tablets.

| | |
|---|---|
| Weight of tablet: | 220 mg |
| Tablet shape: | 10 mm, flat-faced, with bevelled edges. |

### Example 3: Tablets containing 150 mg of active substance by aqueous granulation process

| 1 tablet contains: | |
|---|---|
| active substance | 150.0 mg |
| powdered lactose | 98.0mg |
| corn starch | 40.0 mg |
| colloidal silica | 1.0 mg |
| polyvinylpyrrolidone | 10.0 mg |
| magnesium stearate | 1.0 mg |
| | 300.0 mg |

### Preparation:

The active substance mixed with lactose, corn starch is moistened with a 20% aqueous polyvinylpyrrolidone solution and passed through a screen with a mesh size of 1.5 mm. The granules, dried at 45°C, are passed through the same screen again and mixed with the specified amount of magnesium stearate and colloidal silica. Tablets are pressed from the final blend.

| | |
|---|---|
| Weight of tablet: | 300 mg |
| Tablet shape: | 14 mm x 6.8 mm, oblong biconvex with embossement |

### Example 4: Hard capsules containing 150 mg of active substance in granules Composition:

| 1 capsule contains: | |
|---|---|
| active substance | 150.0 mg |
| microcrystalline cellulose | 80.0 mg |
| lactose (spray-dried) | 87.0 mg |
| colloidal silica | 10.0 mg |
| | 320.0 mg |

### Preparation:

The active substance is mixed with the excipients in a high-shear mixer, passed through a screen with a mesh size of 0.75 mm and homogeneously mixed using a suitable apparatus. The finished mixture is packed into size 1 hard gelatin capsules.

| | |
|---|---|
| Capsule filling: | 320 mg |
| Capsule shape: | size 1, opaque hard capsule. |

### Example 5:

### Suppositories containing 150 mg of active substance

### Composition:

| 1 suppository contains: | |
|---|---|
| active substance | 150.0 mg |
| polyethyleneglycol 1500 | 550.0 mg |
| polyethyleneglycol 6000 | 460.0 mg |
| polyoxyethylene sorbitan monostearate | 840.0 mg |
| | 2,000.0 mg |

### Preparation:

After the suppository mass has been melted the active substance is homogeneously suspended therein and the melt is poured into chilled moulds.

### Example 6: Hard capsules containing 150 mg of active substance in coated pellets and 150 mg of a second active in coated pellets

### Composition:

1 capsule contains:

| | |
|---|---|
| active substance | 150.0 mg |
| active substance 2 | 150.0 mg |
| powdered lactose | 98.0mg |
| corn starch | 40.0 mg |
| polyvinylpyrrolidone | 10.0 mg |
| hydroxypropylmethylcellulose | 7.5 mg |
| polyethylene glycol | 1.0 mg |
| polydextrose | 5.0 mg |
| talc | 1.0 mg |
| water (volatile) | *** |
| | 462.50 mg |

### Preparation:

The active substances are separately extruded with half of the lactose and the corn starch by a wet-extrusion process and rounded in a spheronizer to pellet. Each fraction is dryed in a fluid-bed dryer/coater and subsequently coated with half of a solution of the other excipients. The dryed pellets are homogeneously mixed and packed into size 0 hard capsules.

| | |
|---|---|
| Capsule filling: | 462.5 mg |
| Capsule shape: | size 0, opaque hard capsule. |

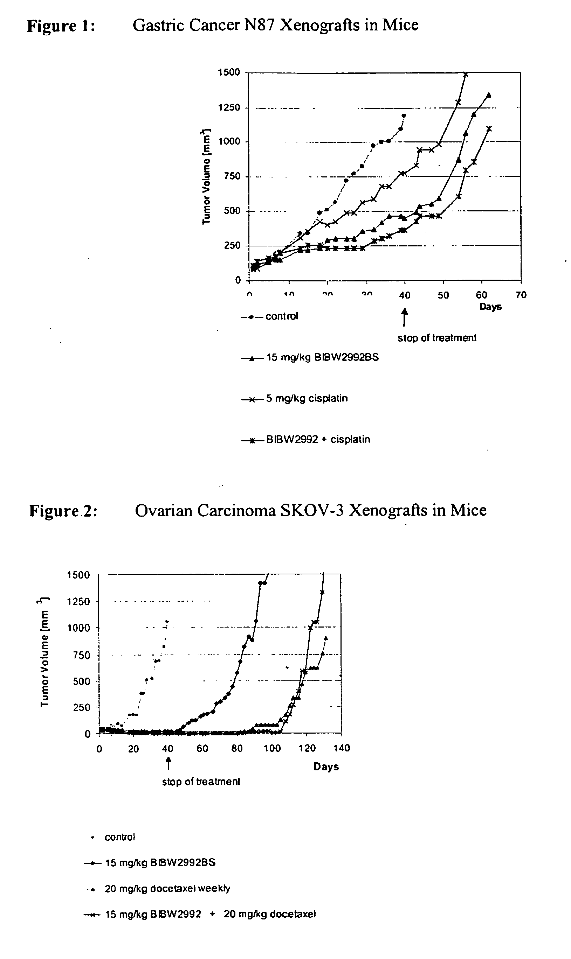

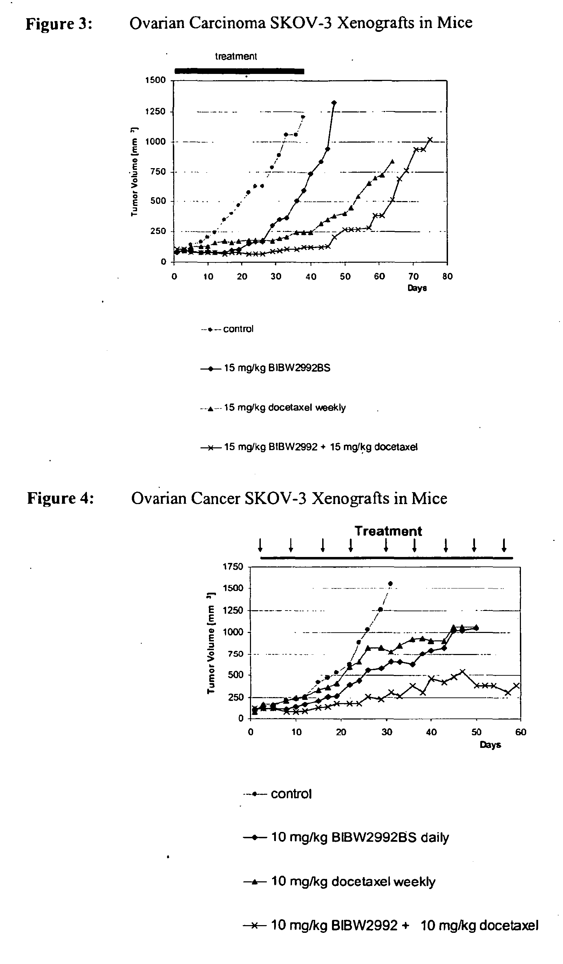

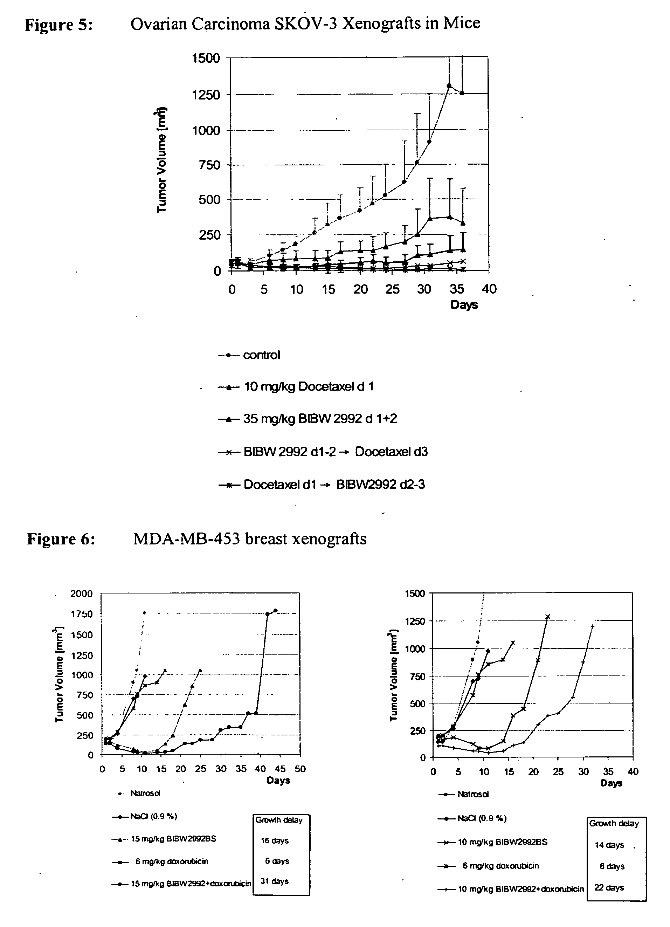

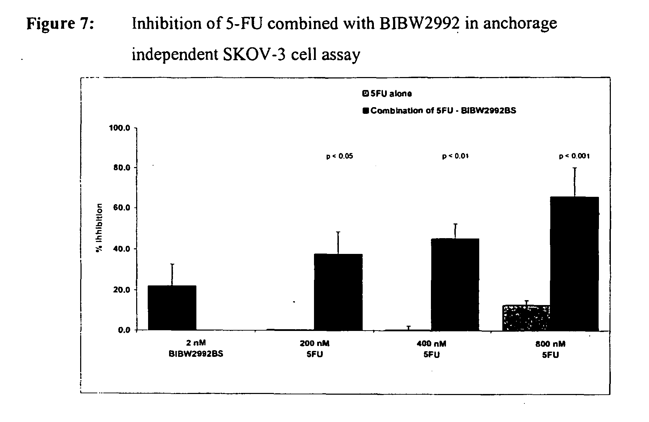

## Claims

1. A pharmaceutical composition (embodiment B) comprising effective amounts of:
as a compound **1**
4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline,
or a pharmacologically acceptable acid addition salt thereof,
and as a chemotherapeutic agent **2** cetuximab.

2. A pharmaceutical composition according to claim 1, in the form of a combined preparation kit for the treatment of cancer diseases, comprising
(i) a first compartment containing a pharmaceutical composition comprising a therapeutically effective amount of compound **1** as defined in claim 1; and
(ii) a second containment containing a pharmaceutical composition comprising a therapeutically effective amount of the further chemotherapeutic agent 2 cetuximab;
said kit being optionally adapted for a co-treatment with radiotherapy or radio-immunotherapy.

3. A pharmaceutical composition according to claim 1 or 2, wherein compound **1** is
4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline dimaleate salt.

4. Use of 4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline for the manufacture of a pharmaceutical composition according to claim 1 (embodiment B) comprising effective amounts of:
4-[(3-chloro-4-fluorophenyl)amino]-6-{[4-(N,N-dimethylamino)-1-oxo-2-buten-1-yl]amino}-7-((S)-tetrahydrofuran-3-yloxy)-quinazoline,
or a pharmacologically acceptable acid addition salt thereof,
and cetuximab,
in the form of a combined preparation for simultaneous, separate or sequential use in the treatment of a patient suffering from cancer selected from the group consisting of
o Head and neck tumours: SCC, AC, transitional cell cancers, mucoepidermoid cancers, undifferentiated carcinomas;
o Colorectal cancers, metastatic or non-metastatic: AC, including hereditary forms of AC, carcinoid, sarcoma;
o Pancreatic cancers: AC, including ductal and acinary cancers, papillary, adenosquamous, undifferentiated, tumours of the endocrine pancreas;
o Breast cancers, metastatic or non-metastatic: AC, including invasive ductal, lobular and medullary cancers, tubular, mucinous cancers, Paget-carcinoma, inflammatory carcinoma, ductal and lobular carcinoma in situ;
o Prostate cancers: AC, small cell, SCC;
o Gastric cancers: AC, adenosquamous, anaplastic;
o Ovarian cancer;
o Non-small cell lung cancers (NSCLC): SCC, spindle cell carcinoma, AC, bronchioalveolar carcinoma, large cell NSCLC, clear cell NSCLC.

5. The use of claim 4, wherein the patient is a pre-selected cancer patient shown to carry a tumor harboring an activating EGFR mutation.

6. The use of claim 5, wherein the EGFR mutation is selected from the group consisting of the L858R point mutation, deletion/insertion mutations in the ELREA sequence, the T790M point mutation in exon 20, and double mutations such as the combined L858R / T790M mutation.

7. The use of claim 4, wherein the patient is a pre-selected cancer patient shown to carry a tumor harboring an activating HER2 mutation.

8. The use of claim 7, wherein the HER2 mutation is the M774_A775insAYVM mutation.

## Patentansprüche

1. Pharmazeutische Zusammensetzung (Ausführungsform B), umfassend wirksame Mengen von:
als Verbindung 1
4-[(3-Chlor-4-fluorphenyl)amino]-6-{[4-(N,N-dimethylamino)-1-oxo-2-buten-1-yl]-amino}-7-((S)-tetrahydrofuran-3-yloxy)chinazolin,
oder ein pharmakologisch akzeptables Säureadditionssalz davon,
und als chemotherapeutisches Mittel **2** Cetuximab.

2. Pharmazeutische Zusammensetzung nach Anspruch 1, in Form eines kombinierten Zubereitungskits zur Behandlung von Krebserkrankungen, umfassend:
(i) ein erstes Behältnis, enthaltend eine pharmazeutische Zusammensetzung, umfassend eine therapeutisch wirksame Menge von Verbindung 1, wie in Anspruch 1 definiert; und
(ii) ein zweites Behältnis, enthaltend eine pharmazeutische Zusammensetzung, umfassend eine therapeutisch wirksame Menge des weiteren chemotherapeutischen Mittels **2** Cetuximab;
wobei das Kit gegebenenfalls angepasst ist fiir eine Co-Behandlung mit Radiotherapie oder Radioimmuntherapie.

3. Pharmazeutische Zusammensetzung nach Anspruch 1 oder 2, wobei die Verbindung **1**
4-[(3-Chlor-4-fluorphenyl)amino]-6-{[4-(N,N-dimethylamino)-1-oxo-2-buten-1-yl]-amino}-7-((S)-tetrahydrofuran-3-yloxy)chinazolin-dimaleatsalz
darstellt.

4. Verwendung von 4-[(3-Chlor-4-fluorphenyl)amino]-6-{[4-(N,N-dimethylamino)-1-oxo-2-buten-1-yl]amino}-7-((S)-tetrahydrofuran-3-yloxy)chinazolin zur Herstellung einer pharmazeutischen Zusammensetzung nach Anspruch 1 (Ausführungsform B), umfassend wirksame Mengen von:
4-[(3-Chlor-4-fluorphenyl)amino]-6-{[4-(N,N-dimethylamino)-1-oxo-2-buten-1-yl]-amino}-7-((S)-tetrahydrofuran-3-yloxy)chinazolin,
oder ein pharmakologisch akzeptables Säureadditionssalz davon,
und Cetuximab,
in Form einer kombinierten Zubereitung zur gleichzeitigen, getrennten oder aufeinanderfolgenden Verwendung bei der Behandlung eines Patienten, der an Krebs leidet, ausgewählt aus der Gruppe, bestehend aus
o Kopf- und Nackentumoren: SCC, AC, Übergangszellkrebs, Mukoepidermoidkrebs, undifferenzierte Karzinome;
o Kolorektalkrebs, metastasierend oder nicht-metastasierend: AC, einschließlich hereditärer Formen von AC, karzinoid, Sarkom;
o Pankreaskrebs: AC, einschließlich duktalem und acinärem Krebs, papillär, adenosquamös, undifferenziert, Tumoren der endokrinen Pankreas;
o Brustkrebs, metastasierend oder nicht-metastasierend: AC, einschließlich invasiv duktalem, lobulärem und medullärem Krebs, tubulärem, muzinösem Krebs, Paget-Karzinom, entzündlichem Karzinom, duktalem und lobulärem Karzinom in situ;
o Prostatakrebs: AC, kleinzellig, SCC;
o Magenkrebs: AC, adenosquamös, anaplastisch;
o Eierstockkrebs;
o Nicht-kleinzelliger Lungenkrebs (NSCLC): SCC, Spindelzellenkarzinom, AC, Bronchoalveolärkarzinom, großzelliger NSCLC, klarzelliger NSCLC.

5. Verwendung nach Anspruch 4, wobei der Patient ein vorausgewählter Krebspatient ist und zeigt, dass er einen Tumor hat, der eine aktivierende EGFR-Mutation aufweist.

6. Verwendung nach Anspruch 5, wobei die EGFR-Mutation ausgewählt ist aus der Gruppe, bestehend aus der L858R-Punktmutation, Deletions-/Insertions-Mutationen in der ELREA-Sequenz, der T790M-Punktmutation in Exon 20 und Doppelmutationen, wie der kombinierten L858R/T790M-Mutation.

7. Verwendung nach Anspruch 4, wobei der Patient ein vorausgewählter Krebspatient ist, und zeigt, dass er einen Tumor hat, der eine aktivierende HER2-Mutation aufweist.

8. Verwendung nach Anspruch 7, wobei die HER2-Mutation die M774_A775insAYVM-Mutation darstellt.

## Revendications

1. Composition pharmaceutique (mode de réalisation B) comprenant des quantités efficaces de :
en tant que composé **1**
la 4-[(3-chloro-4-fluorophényl)amino]-6-{[4-(N,N-diméthylamino)-1-oxo-2-butén-1-yl]amino}-7-((S)-tétrahydrofuran-3-yloxy)-quinazoline,
ou un sel d'addition d'acide pharmacologiquement acceptable de celle-ci,
et en tant qu'agent chimiothérapeutique **2**, le cétuximab.

2. Composition pharmaceutique selon la revendication 1, sous la forme d'un kit de préparation combinée destiné au traitement de maladies cancéreuses, comprenant
(i) un premier compartiment contenant une composition pharmaceutique comprenant une quantité thérapeutiquement efficace de composé **1** tel que défini dans la revendication 1 ; et
(ii) un second confinement contenant une composition pharmaceutique comprenant une quantité thérapeutiquement efficace de l'autre agent chimiothérapeutique **2**, le cétuximab ;
ledit kit étant éventuellement adapté pour un cotraitement avec une radiothérapie ou une radio-immunothérapie.

3. Composition pharmaceutique selon la revendication 1 ou 2, dans laquelle le composé **1** est
le sel dimaléate de 4-[(3-chloro-4-fluorophényl)-amino]-6-{[4-(N,N-diméthylamino)-1-oxo-2-butén-1-yl]amino}-7-((S)-tétrahydrofuran-3-yloxy)-quinazoline.

4. Utilisation de 4-[(3-chloro-4-fluorophényl)amino]-6-{[4-(N,N-diméthylamino)-1-oxo-2-butén-1-y1]amino}-7-((S)-tétrahydrofuran-3-yloxy)-quinazoline pour la fabrication d'une composition pharmaceutique selon la revendication 1 (mode de réalisation B) comprenant des quantités efficaces de :
4-[(3-chloro-4-fluorophényl)amino]-6-{[4-(N,N-diméthylamino)-1-oxo-2-butén-1-yl]amino}-7-((S)-tétrahydrofuran-3-yloxy)-quinazoline,
ou d'un sel d'addition d'acide pharmacologiquement acceptable de celle-ci,
et de cétuximab,
sous la forme d'une préparation combinée pour une utilisation simultanée, séparée ou séquentielle dans le traitement d'un patient souffrant d'un cancer choisi dans le groupe constitué des
o tumeurs de la tête et du cou : SCC, AC, cancers à cellules transitionnelles, cancers muco-épidermoïdes, carcinomes indifférenciés ;
o cancers colorectaux, métastasés ou non métastasés : AC, y compris des formes héréditaires d'AC, carcinoïdes, sarcomes ;
o cancers pancréatiques : AC, y compris des cancers canalaires et acineux, papillaires, adénosquameux, indifférenciés, des tumeurs du pancréas endocrine ;
o cancers du sein, métastasés ou non métastasés : AC, y compris des cancers canalaires, lobulaires et médullaires invasifs, tubulaires, des cancers mucineux, des carcinomes de Paget, des carcinomes inflammatoires, des carcinomes canalaires et lobulaires *in situ ;*
o cancers de la Prostate AC, à petites cellules, SCC ;
o cancers gastriques : AC, adénosquameux, anaplasiques ;
o cancer ovarien ;
o cancers du poumon non à petites cellules (NSCLC) : SCC, carcinomes à cellules fusiformes, AC, carcinomes bronchio-alvéolaires, NSCLC à grandes cellules, NSCLC à cellules claires.

5. Utilisation selon la revendication 4, où le patient est un patient cancéreux présélectionné qui a été démontré comme étant porteur d'une tumeur abritant une mutation activatrice de l'EGFR.

6. Utilisation selon la revendication 5, où la mutation de l'EGFR est choisie dans le groupe consistant en la mutation ponctuelle L858R, des mutations par délétion/ insertion dans la séquence ELREA, la mutation ponctuelle T790M dans l'exon 20, et des mutations doubles comme la mutation combinée L858R / T790M.

7. Utilisation selon la revendication 4, où le patient est un patient cancéreux présélectionné qui a été démontré comme étant porteur d'une tumeur abritant une mutation activatrice de HER2.

8. Utilisation selon la revendication 7, où la mutation de HER2 est la mutation M774_A775insAYVM.
